# EUROPÄISCHE PATENTSCHRIFT

(11) **EP 0 405 001 B1**
(45) Veröffentlichungstag und Bekanntmachung des Hinweises auf die Patenterteilung: **09.11.1994**
(21) Anmeldenummer: 89111995.0
(22) Anmeldetag: 30.06.1989
(51) Int. Cl.: A61B 6/04

(54) **Patientenlagerungstisch mit einer mit einem Ausschnitt versehenen Lagerungsplatte und einem die Lagerungsplatte tragenden Basisteil**
Patient-supporting table having a supporting plate provided with a cut and a base carrying the supporting plate
Table de support pour patient à plateau de support muni d'une découpure et à base supportant le plateau de support

(43) Veröffentlichungstag der Anmeldung: 02.01.1991
(73) Patentinhaber: SIEMENS AKTIENGESELLSCHAFT, 80333 München (DE)
(72) Erfinder: Schäfer, Willi, Dipl.-Ing.(FH), D-8520 Erlangen (DE)

(56) Entgegenhaltungen:
- EP-A- 0 255 645
- EP-A- 0 294 679
- DE-A- 2 759 079
- FR-A- 2 013 178
- US-A- 4 568 071

## Beschreibung

Die Erfindung betrifft einen Patientenlagerungstisch mit einer Lagerungsplatte, welche eine Auflagefläche für einen Patienten aufweist und mit einem Ausschnitt versehen ist, durch den die Körperoberfläche eines auf der Auflagefläche liegenden Patienten zu medizinischen Zwecken zugänglich ist, und mit einem die Lagerungsplatte tragenden Basisteil, wobei die Lagerungsplatte relativ zu dem Basisteil in einer Verstellrichtung verstellbar ist.

Solche Patientenlagerungstische werden für die unterschiedlichsten Anwendungen benötigt, bei denen es erforderlich ist, daß die Körperoberfläche eines Patienten durch den Ausschnitt zugänglich ist, sei es um einen Eingriff vornehmen zu können oder um ein medizinisches Gerät, z.B. einen Lithotripter, an die Körperoberfläche des Patienten applizieren zu können, und die Lagerungsplatte relativ zu dem Basisteil verstellbar ist, um den Patienten wie gewünscht positionieren zu können.

Ein solcher Patientenlagerungstisch ist in dem Prospekt der Fa. Siemens "LITHOSTAR Universeller urologischer Arbeitsplatz für Therapie und Diagnostik", Druckzeichen: A91001-M1027-G490-01 PA 4864, beschrieben. Bei dem bekannten Patientenlagerungstisch ist ein unterhalb der Lagerungsplatte angeordnetes Stoßwellenrohr vorgesehen, das zur Zertrümmerung von im Körper des Patienten befindlichen Konkrementen, z.B. Nierensteinen, fokussierte Stoßwellen abgibt. Zur Behandlung wird das Stoßwellenrohr durch den als rechteckige Öffnung ausgeführten Ausschnitt der Lagerungsplatte mit der Körperoberfläche eines auf der Lagerungsplatte liegenden Patienten in Eingriff gebracht und derart akustisch an die Körperoberfläche des Patienten angekoppelt, daß sich das zu zertrümmernde Konkrement im Brennpunkt der Stoßwellen befindet. Unter der Einwirkung einer Serie von mittels des Stoßwellenrohres erzeugter fokussierter Stoßwellen zerfällt das Konkrement in Bruchstücke, die auf natürlichem Wege abgehen können. Um das zu zertrümmernde Konkrement in den Brennpunkt der Stoßwellen bringen zu können, ist eine exakte Positionierung des Patienten relativ zu dem Stoßwellenrohr erforderlich. Bei dem bekannten Patientenlagerungstisch besteht deshalb die Möglichkeit, die Lagerungsplatte samt des darauf liegenden Patienten relativ zu dem Basisteil und dem Stoßwellenrohr in Tischlängsrichtung und quer dazu motorisch zu verschieben. Um ein Durchfallen des unter Umständen sedierten oder sogar narkotisierten Patienten durch die Öffnung zu verhindern, weist diese jedoch in Tischlängsrichtung nur relativ geringe Abmessungen auf. Da außerdem während des Verschiebens der Lagerungsplatte das Stoßwellenrohr in deren Öffnung angeordnet ist, kann die Lagerungsplatte relativ zu dem Stoßwellenrohr nur um ein geringes Maß in Tischlängsrichtung verschoben werden. Andernfalls würden die Begrenzungskanten der Öffnung an dem Stoßwellenrohr anstoßen und dieses aus seiner vorbestimmten Lage verschieben oder gar beschädigen. Bei dem bekannten Patientenlagerungstisch müssen deshalb die behandelnden Ärzte und das Bedienungspersonal den Patienten wenigstens in Tischlängsrichtung zunächst manuell schon so genau auf der Lagerungsplatte ausrichten, daß für dessen exakte motorische Positionierung der durch die Abmessungen des Stoßwellenrohres und der Öffnung begrenzte Verstellbereich der Lagerungsplatte ausreicht. Dies ist infolge des Umstandes, daß der Patient sediert oder narkotisiert ist, mit erheblicher körperlicher Anstrengung für die behandelnden Ärzte und das Bedienungspersonal verbunden. Außerdem besteht die Gefahr, daß ein Patient, der z.B. in Querrichtung des Patientenlagerungstisches bereits exakt positioniert ist, wieder verschoben wird. Des weiteren ist die beschriebene Vorgehensweise für den Patienten wenig schonend, da erhebliche Kräfte auf diesen ausgeübt werden.

Eine gewisse Verbesserung kann hier ein in der EP-A-0 294 679 beschriebene Patientenlagerungstisch bringen, dessen Lagerungsplatte einen Ausschnitt aufweist, der durch zwei unabhängig voneinander gegenläufig verstellbare Schieber ganz oder teilweise verschließbar ist.

Ein in der FR-A-2 013 178 beschriebene Patientenlagerungstisch weist eine Lagerungsplatte auf, die in zwei durch ein Gestänge miteinander verbundene Abschnitte unterteilt ist, deren einander zugewandte Kanten einen durchgehenden Spalt begrenzen, der mittels eines Schiebers ganz oder teilweise verschließbar ist.

Sowohl im Falle der EP-A-0 294 679 als auch der FR-A-2 013 178 ist eine Verstellbarkeit der Lagerungsplatte relativ zu einem Basisteil nicht vorgesehen. Diese Maßnahme ist jedoch in der US-A-4 568 071 für einen Patientenlagerungstisch beschrieben, dessen Lagerungsplatte keinerlei Ausschnitt aufweist, deren Unterseite jedoch der Oberseite eines unterhalb der Lagerungsplatte angebrachten Basisteils zugewandt ist.

Der Erfindung liegt die Aufgabe zugrunde, einen Patientenlagerungstisch der eingangs genannten Art so auszubilden, daß auf einfache Weise eine exakte Positionierung des Patienten relativ zu dem Ausschnitt möglich ist, ohne daß dies mit körperlicher Anstrengung für die behandelnden Ärzte und das Bedienungspersonal verbunden ist, ohne daß nennenswerte Kräfte auf den Patienten ausgeübt werden müssen und ohne daß die Gefahr eines Durchrutschens des Patienten durch den Ausschnitt besteht.

Nach der Erfindung wird diese Aufgabe dadurch gelöst, daß
a) das Basisteil unterhalb der Lagerungsplatte angeordnet ist und eine Oberseite aufweist, die der Unterseite der Lagerungsplatte zugewandt ist,
b) das Basisteil eine sich durch dessen Oberseite erstreckende Öffnung aufweist, deren Erstreckung zumindest in Verstellrichtung geringer ist als die entsprechende Erstreckung des Ausschnittes der Lagerungsplatte,
c) die Lagerungsplatte relativ zu dem Basisteil derart positionierbar ist, daß der Ausschnitt der Lagerungsplatte die Öffnung des Basisteiles freigibt, und
d) die Oberseite des Basisteiles innerhalb des Ausschnittes der Lagerungsplatte eine zusätzliche Auflagefläche bildet.

Um den Zugang zur Körperoberfläche eines auf der Lagerungsplatte liegenden Patienten zu ermöglichen, wird also die Lagerungsplatte relativ zu dem Basisteil derart positioniert, daß der Ausschnitt der Lagerungsplatte die Öffnung des Basisteiles freigibt. Infolge des Umstandes, daß das Basisteil eine plattenförmige Oberfläche aufweist, die im Bereich des Ausschnittes der Lagerungsplatte die Auflagefläche bildet und infolge des Umstandes, daß die Öffnung des Basisteiles in Verstellrichtung eine Erstreckung aufweist, die geringer ist als die des Ausschnittes der Lagerungsplatte, erfährt ein auf der Lagerungsplatte liegender Patient durch vorzugsweise plattenförmige die Oberseite des Basisteiles im Bereich des Ausschnittes eine zusätzliche Unterstützung. Dabei ist es im Sinne einer wirksamen Unterstützung des Patienten zweckmäßig, wenn die Oberfläche des Basisteiles nur einen geringen Abstand von der Auflagefläche aufweist. Die Abmessungen der Öffnung des Basisteiles sind derart gewählt, daß ein dem jeweiligen medizinischen Zweck entsprechender Bereich der Körperoberfläche eines auf der Lagerungsplatte liegenden Patienten zugänglich ist. Die Abmessungen des Ausschnittes sind dagegen zumindest in Verstellrichtung so bemessen, daß es ohne besondere Sorgfalt möglich ist, einen Patienten derart auf der Lagerungsplatte zu positionieren, daß sich derjenige Bereich seiner Körperoberfläche, der für den jeweiligen medizinischen Zweck zugänglich sein muß, innerhalb des Ausschnittes befindet. Durch Verstellen der Lagerungsplatte relativ zu dem Basisteil wird dann der relevante Bereich der Körperoberfläche des Patienten über die Öffnung des Basisteiles gebracht. Es genügt also, den Patienten auf der Lagerungsplatte grob vorzupositionieren, um ihn dann, ohne daß dies mit besonderen körperlichen Anstrengungen für die behandelnden Ärzte und das Bedienpersonal verbunden ist, durch Verstellen der Lagerungsplatte relativ zu dem Basisteil exakt positionieren zu können. Auch für den Patienten gestaltet sich der Positionierungsvorgang schonend, da nur geringe Kräfte auf ihn ausgeübt werden. Ein Durchrutschen des Patienten durch den Ausschnitt bzw. die Öffnung ist ausgeschlossen. Die Erfindung eignet sich besonders für solche Patientenlagerungstische, deren Lagerungsplatte relativ zu dem Basisteil geradlinig, vorzugsweise wenigstens in Richtung der Längsachse des Patientenlagerungstisches, verstellbar ist.

Eine besonders gute zusätzliche Unterstützung des Patienten im Bereich des Ausschnittes ergibt sich, wenn die Lagerungsplatte wenigstens mit den quer zur Verstellrichtung verlaufenden Begrenzungskanten des Ausschnittes im wesentlichen spaltlos an der Oberseite des Basisteiles anliegt. Durch diese Maßnahme ist zugleich die Gefahr vermindert, daß beim Verstellen der Lagerungsplatte Hautfalten des Patienten zwischen der Lagerungsplatte und dem Basisteil eingequetscht werden. Diese Gefahr läßt sich weiter vermindern, wenn die Auflagefläche der Lagerungsplatte mit einer Polsterauflage versehen ist, die bis an die Begrenzungskanten des Ausschnittes reicht, da in diesem Falle ein Anliegen der Körperoberfläche des Patienten an der Oberseite des Basisteiles unmittelbar im Bereich der Begrenzungskanten des Ausschnittes vermieden ist.

Um im Interesse einer guten zusätzlichen Unterstützung des Patienten durch die Oberseite des Basisteiles einen zu großen Abstand zwischen der Auflagefläche der Lagerungsplatte und der Oberfläche des Basisteiles zu vermeiden, ist es vorteilhaft, wenn gemäß einer Ausführungsform der Erfindung die Lagerungsplatte als dünnwandiges Bauteil ausgeführt ist.

Eine besonders vorteilhafte Ausführungsform der Erfindung sieht vor, daß die der Auflagefläche abgewandte Unterseite der Lagerungsplatte als Eingriffsfläche ausgebildet ist, die an einer entsprechend geformten Eingriffsfläche des Basisteiles flächenhaft anliegt, daß die Eingriffsflächen sich zu den quer zur Verstellrichtung verlaufenden Begrenzungskanten des Ausschnittes bzw. der Öffnung erstrecken, und daß die Eingriffsflächen der Lagerungsplatte und des Basisteiles beim Verstellen der Lagerungsplatte relativ zum Basisteil aufeinander gleiten. Auf diese Weise ist zum einen insbesondere für den Fall, daß die Lagerungsplatte als dünnwandiges Bauteil ausgeführt ist, sichergestellt, daß die Lagerungsplatte eine ausreichende Unterstützung durch das Basisteil erfährt. Zum anderen ist ohne zusätzlichen Aufwand gewährleistet, daß die Oberseite des Basisteiles den zur zusätzlichen Unterstützung des Patienten im Bereich des Ausschnittes erforderlichen geringen Abstand von der Auflagefläche der Lagerungsplatte aufweist und die Lagerungsplatte im Bereich des Ausschnittes im wesentlichen spaltlos an der Oberseite des Basisteiles anliegt.

Gemäß einer Variante der Erfindung ist vorgesehen, daß der Ausschnitt als Ausnehmung in einer der Begrenzungskanten der Lagerungsplatte ausgeführt ist und die Öffnung als Ausnehmung in der entsprechenden Begrenzungskante des Basisteiles ausgeführt ist. Hierdurch ergibt sich der Vorteil, daß die Körperoberfläche des Patienten nicht nur direkt von unten sondern auch von unten seitlich gut zugänglich ist. Die Ausbildung der Öffnung des Basisteiles als Ausnehmung ist in technisch einfacher Weise realisierbar, wenn das Basisteil zwei Plattenteile aufweist, die in Verstellrichtung gesehen auf unterschiedlichen Seiten der Öffnung angeordnet sind, und wenn die Plattenteile durch einen Tragholm miteinander verbunden sind, der sich wenigstens im Bereich der Ausnehmung der Lagerungsplatte entlang der der Ausnehmung gegenüberliegenden Begrenzungskante der Lagerungsplatte erstreckt. Dabei dient der Tragholm nicht nur zur Verbindung der beiden Plattenteile des Basisteiles, sondern bewirkt außerdem eine wirksame Unterstützung und Versteifung der Lagerungsplatte.

Gemäß einer Ausführungsform der Erfindung ist vorgesehen, daß die Lagerungsplatte an dem Basisteil wahlweise derart anbringbar ist, daß sich die Ausnehmung auf der einen oder der anderen Seite des Patientenlagerungstisches befindet. Da Patientenlagerungstische in der Regel ein definiertes Fuß- und ein definiertes Kopfende aufweisen, ist durch diese Maßnahme gewährleistet, daß für beide Körperseiten des Patienten die durch die Ausbildung des Ausschnittes als Ausnehmung verbesserte Zugänglichkeit nutzbar ist. Der gleiche Vorteil wird auch erreicht, wenn die Lagerungsplatte zweiteilig ausgeführt ist, wobei ein Plattenabschnitt die Ausnehmung aufweist und mit dem anderen Plattenabschnitt wahlweise derart verbindbar ist, daß sich die Ausnehmung auf der einen oder der anderen Seite des Patientenlagerungstisches befindet. In diesem Falle ergibt sich zusätzlich der Vorteil, daß zur Änderung der Position der Ausnehmung nicht die gesamte Lagerungsplatte, sondern lediglich der mit der Ausnehmung versehene Plattenabschnitt gewendet werden muß, was erheblich leichter zu bewerkstelligen ist.

Für den Fall, daß die Lagerungsplatte derart wendbar ist, daß sich die Ausnehmung auf der einen oder der anderen Seite des Patientenlagerungstisches befinden kann, sind gemäß einer Ausführungsform der Erfindung zwei Tragholme vorgesehen, die in Abhängigkeit von der Lage der Ausnehmung auf der einen oder der anderen Seite des Patientenlagerungstisches wahlweise ausziehbar sind, so daß unabhängig von der Position der Ausnehmung auf der einen oder anderen Seite des Patientenlagerungstisches eine sichere Unterstützung der Lagerungsplatte gewährleistet ist und zugleich die Ausnehmung des Basisteiles der Ausnehmung der Lagerungsplatte entsprechend orientiert ist.

Ausführungsbeispiele der Erfindung sind in den beigefügten Zeichnungen dargestellt. Es zeigen:
- Fig. 1: eine perspektivische Ansicht eines erfindungsgemäßen Patientenlagerungstisches,
- Fig. 2: einen Querschnitt durch den Patientenlagerungstisch entsprechend der Linie II-II in Fig. 1,
- Fig. 3: einen Längsschnitt durch den Patientenlagerungstisch entsprechend der Linie III-III in Fig. 1,
- Fig. 4: eine perspektivische Darstellung der Einzelheit A gemäß Fig. 1 bei abgenommener Lagerungsplatte,
- Fig. 5: eine perspektivische Ansicht des Patientenlagerungstisches gemäß Fig. 1 bei gewendeter Lagerungsplatte,
- Fig. 6: eine perspektivische Ansicht eines weiteren erfindungsgemäßen Patientenlagerungstisches, und
- Fig. 7: eine perspektivische Ansicht der Einzelheit B gemäß Fig. 6 in Form einer Explosionsdarstellung.

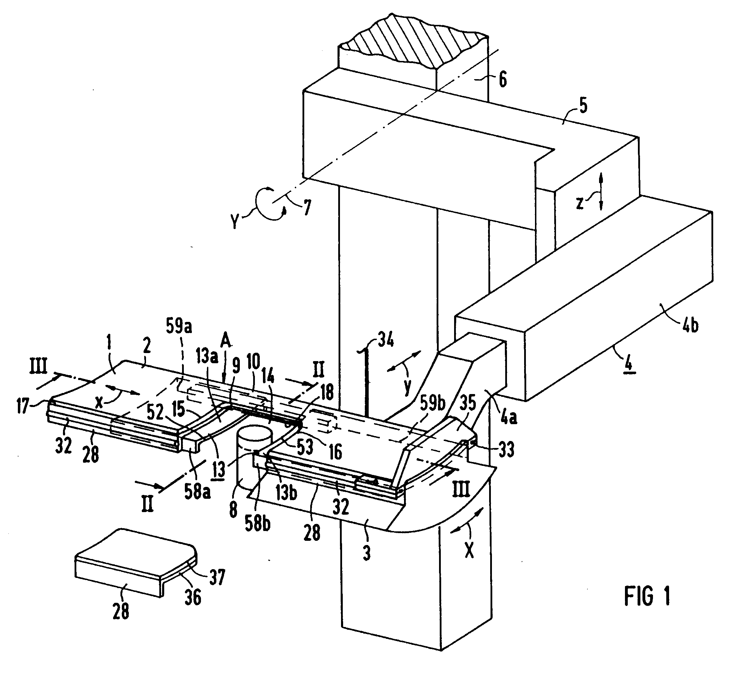

Die Fig. 1 bis 5 zeigen einen erfindungsgemäßen Patientenlagerungstisch, der Bestandteil eines Arbeitsplatzes zur Behandlung eines Patienten mit fokussierten Stoßwellen, beispielsweise zur nicht-invasiven Zertrümmerung von im Körper des Patienten befindlichen Konkrementen wie Nieren- oder Gallenblasensteinen, ist. Die fokussierten Stoßwellen werden mittels einer Stoßwellenquelle, wie sie beispielsweise in der DE-OS 33 28 051 beschrieben ist, erzeugt und in den Körper eines zu behandelnden Patienten eingeleitet, indem die Stoßwellenquelle mit ihrem durch einen flexiblen Balg gebildeten Applikationsende mit der Körperoberfläche des Patienten derart in Eingriff gebracht wird, daß eine akustische Koppelung vorliegt. Dabei werden der Patient und die Stoßwellenquelle durch entsprechendes Verstellen des Patientenlagerungstisches und der Stoßwellenquelle relativ zueinander derart positioniert, daß sich das zu zertrümmernde Konkrement im Fokus der Stoßwellen befindet. Unter der Wirkung einer Vielzahl von Stoßwellen zerfällt das Konkrement in feine Bruchstücke, die auf natürlichem Wege vom Patienten ausgeschieden werden können.

Der Patientenlagerungstisch besitzt eine Lagerungsplatte 1 mit einer Auflagefläche 2 für den Patienten. Außerdem ist ein Basisteil 3 vorgesehen, das die Lagerungsplatte 1 trägt, wobei die Lagerungsplatte 1 von dem Basisteil 3 getrennt werden kann.

Der Patientenlagerungstisch ist mit seinem Basisteil 3 derart mittels geeigneter, nicht dargestellter Gelenkmittel an einem Tragteil 4 angebracht, daß er um eine parallel zu seiner Längsachse verlaufende Achse schwenkbar ist, so wie dies durch den gekrümmten Doppelpfeil X angedeutet ist. Das Tragteil 4 weist zwei teleskopartig ineinander gefügte Armteile 4a, 4b auf, so daß der Patientenlagerungstisch quer zur Richtung seiner Längsachse in einer parallel zur Auflagefläche 2 verlaufenden Ebene in Richtung des Doppelpfeiles y verstellbar ist. Das Tragteil 4 ist mit seinem Armteil 4b an einem armförmigen Träger 5 mittels geeigneter, nicht dargestellter Führungsmittel derart befestigt, daß der Patientenlagerungstisch rechtwinklig zur Ebene seiner Lagerungsplatte 1 verstellbar ist, so wie dies durch den Doppelpfeil z angedeutet ist. Der Träger 5 ist an einem Ständer 6 um eine quer zur Längsachse des Patientenlagerungstisches und parallel zur Ebene der Lagerungsplatte 1 verlaufende Achse 7 mittels eines geeigneten, nicht dargestellten Gelenkes in Richtung des Doppelpfeiles Y schwenkbar. Außerdem ist die Lagerungsplatte 1 relativ zu dem Basisteil 3 in Richtung der Längsachse des Patientenlagerungstisches geradlinig verstellbar, so wie dies durch den Doppelpfeil x angedeutet ist.

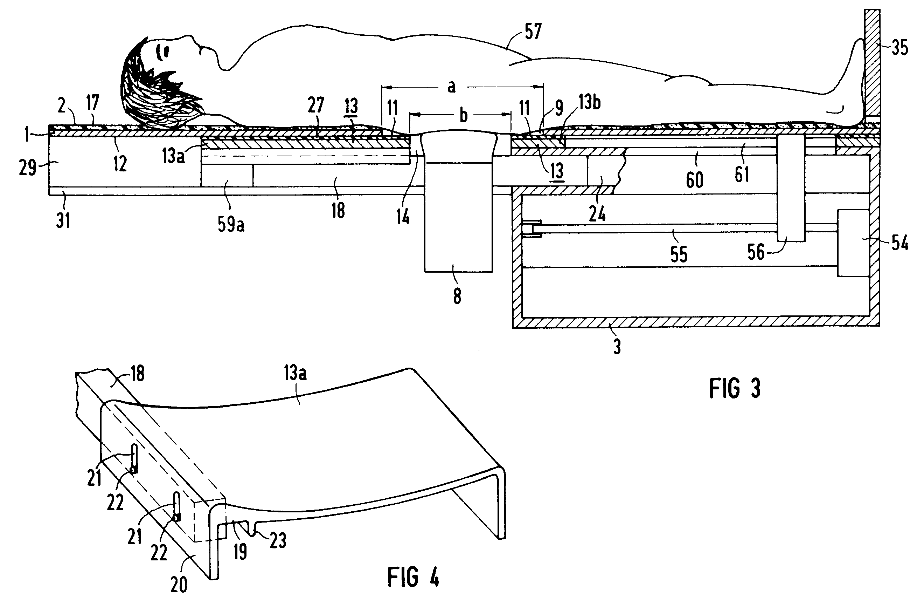

Um die beschriebenen Verstellbewegungen ausführen zu können, sind nicht dargestellte Elektromotore und geeignete Getriebe vorgesehen, die von einer Bedienperson mittels einer ebenfalls nicht dargestellten Bedieneinheit betätigbar sind. Lediglich die zum Verstellen der Lagerungsplatte 1 relativ zu dem Basisteil 3 in Richtung der Längsachse des Patientenlagerungstisches (Doppelpfeil x) vorgesehene Antriebseinrichtung ist in Fig. 3 schematisch dargestellt. Sie besteht aus einem in dem Basisteil 3 angeordneten Elektromotor 54, der eine Gewindespindel 55 antreibt. Die Gewindespindel 55 wirkt mit einer Mutter 56 zusammen, die an der Lagerungsplatte 1 in nicht dargestellter Weise lösbar befestigt ist. Dabei ist in dem Basisteil 3 ein Schlitz 60 vorgesehen, durch den die Mutter 56 mit der Lagerungsplatte 1 in Eingriff steht.

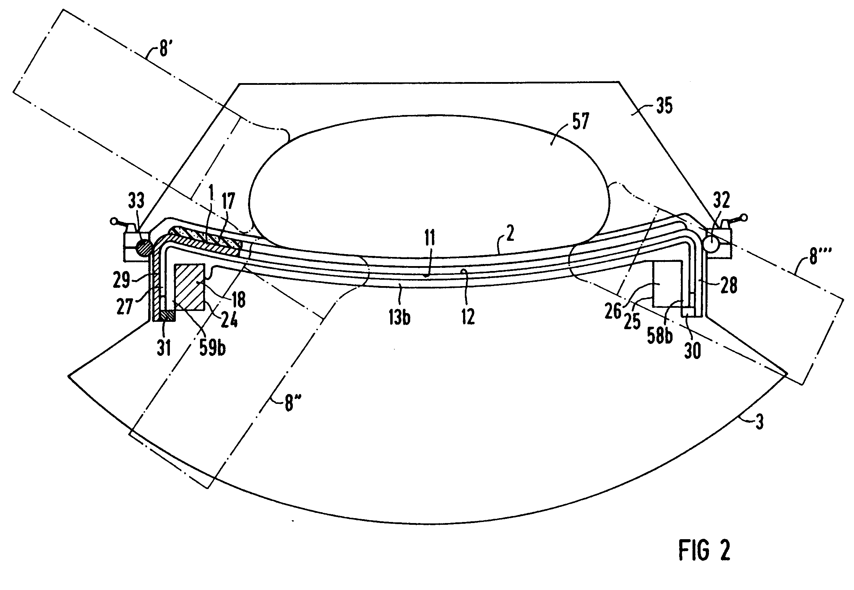

Aus den Fig. 1, 3 und 5 wird deutlich, daß der Patientenlagerungstisch mit seinem Basisteil 3 derart an dem Tragteil 4 angebracht ist, daß der Patientenlagerungstisch nur an seinem einen Ende unterstützt ist. Bei dem unterstützten Ende des Patientenlagerungstisches handelt es sich um dessen Fußende. Das freie Ende des Patientenlagerungstisches bildet dessen Kopfende. Infolge des Umstandes, daß der Patientenlagerungstisch nur an seinem einen Ende unterstützt ist, ergibt sich eine verbesserte Zugänglichkeit zu dem Patientenlagerungstisch bzw. einem auf diesem liegenden Patienten. Um eine an einer geeigneten, nicht dargestellten Halterung angebrachte Stoßwellenquelle 8, die in Fig. 1 schematisch angedeutet ist, aus möglichst beliebigen Richtungen an der Körperoberfläche eines auf der Lagerungsplatte 1 liegenden Patienten zur Anlage bringen zu können, ist die Lagerungsplatte 1 mit einem Ausschnitt versehen, der als etwa rechteckige Ausnehmung 9 in der in Fig. 1 vorderen Längskante der Lagerungsplatte 1 ausgeführt ist. Wie aus Fig. 2 anhand der strichliert dargestellten und mit 8′ und 8˝ bezeichneten Extrempositionen der Stoßwellenquelle 8 ersichtlich ist, ist nur ein relativ geringer Winkelbereich durch den im Bereich der Ausnehmung 9 verbleibenden Abschnitt 10 der Lagerungsplatte 1 versperrt. Insbesondere ist es möglich, die Stoßwellenquelle 8 von schräg unten lateral an den im Querschnitt angedeuteten Körper 57 eines auf der Lagerungsplatte 1 liegenden Patienten anzukoppeln. Diese Position der Stoßwellenquelle 8 ist ebenfalls strichliert dargestellt und mit 8‴ bezeichnet.

Um in Behandlungsfällen, die es erfordern, daß die Stoßwellenquelle 8 von der Unterseite des Patientenlagerungstisches her durch die Ausnehmung 9 mit dem Körper 57 des Patienten in Eingriff gebracht wird, eine Positionierung des Patienten relativ zu der Stoßwellenquelle 8 durch Verschieben der Lagerungsplatte 1 in Richtung der Längsachse des Patientenlagerungstisches bei bereits in der Ausnehmung 9 befindlicher Stoßwellenquelle 8 zu ermöglichen, weist die Ausnehmung 9 in Richtung der Längsachse des Patientenlagerungstisches eine lichte Weite a (Fig. 3) auf, die größer ist als dies die entsprechende Abmessung der Stoßwellenquelle 8, z.B. deren Durchmesser, erfordert. Es reicht zwar aus, wenn die Lagerungsplatte 1 bei in der Ausnehmung 9 befindlicher Stoßwellenquelle 8 in Richtung der Längsachse des Patientenlagerungstisches um etwa ± 100 mm verschiebbar ist; bei einem Durchmesser der Stoßwellenquelle von ca. 250 mm bedeutet dies jedoch, daß die Lagerungsplatte 1 allein keine ausreichende Unterstützung des Patienten im Bereich der dann eine lichte Weite a von ca. 450 mm aufweisenden Ausnehmung 9 bieten kann.

Um eine ausreichende Unterstützung des Patienten im Bereich der Ausnehmung 9 zu gewährleisten und insbesondere ein Durchrutschen des Patienten durch die Ausnehmung 9 zu vermeiden, weist das Basisteil 3 einen der Gestalt der Lagerungsplatte 1 im wesentlichen angepaßten plattenförmigen Abschnitt 13 auf, der derart unterhalb der Lagerungsplatte 1 angeordnet ist, daß seine Oberseite 11 der Unterseite 12 der Lagerungsplatte 1 zugewandt ist. Ähnlich wie die Lagerungsplatte 1 besitzt auch das Basisteil 3 einen Ausschnitt. Dieser ist ähnlich wie der Ausschnitt im Falle der Lagerungsplatte 1 als Ausnehmung 14 in der in Fig. 1 vorderen Längskante des plattenförmigen Abschnittes 13 ausgeführt. Die lichte Weite b der Ausnehmung 14 in Richtung der Längsachse des Patientenlagerungstisches ist wenig größer als der Durchmesser der Stoßwellenquelle 8 und ist somit deutlich kleiner als die lichte Weite a der Ausnehmung 9 der Lagerungsplatte 1.

Es versteht sich, daß bei von der Unterseite des Patientenlagerungstisches her mit dem Körper 57 eines Patienten in Eingriff stehender Stoßwellenquelle 8 die Lagerungsplatte 1 und das Basisteil 3 relativ zueinander so angeordnet sein müssen, daß die Ausnehmung 9 der Lagerungsplatte 1 die Ausnehmung 14 des plattenförmigen Abschnittes 13 vollständig freigibt, so wie dies in den Fig. 1 bis 3 und 5 dargestellt ist. Da die Lagerungsplatte 1, so wie dies aus den Fig. 1 bis 3 ersichtlich ist, mit den quer zur Längsachse des Patientenlagerungstisches und damit quer zur Verschieberichtung verlaufenden Begrenzungskanten 15 und 16 ihrer Ausnehmung 9 im wesentlichen spaltlos an der Oberseite 11 des plattenförmigen Abschnittes 13 anliegt, bietet die Oberseite 11 des plattenförmigen Abschnittes 13 eine wirksame zusätzliche Unterstützung des Körpers 57 des Patienten, die ein Durchrutschen des Patienten durch die Ausnehmung 9 der Lagerungsplatte 1 ausschließt. Der innerhalb der Ausnehmung 9 der Lagerungsplatte 1 befindliche Teil der Oberseite 11 des plattenförmigen Abschnittes 13 des Basisteiles 3 bildet also eine zusätzliche Auflagefläche für den Körper 57 des Patienten.

Wie aus den Figuren ersichtlich ist, ist die Auflagefläche der Lagerungsplatte mit einer Polsterauflage 17 versehen, die bis an die Begrenzungskanten der Ausnehmung 9 heranreicht. Somit ist, so wie dies aus der Fig. 3 ersichtlich ist, sichergestellt, daß unmittelbar im Bereich der Begrenzungskanten 15 und 16 der Ausnehmung 9 der Lagerungsplatte 1 ein Abstand der Körperoberfläche des Patienten von der Oberseite 1 des plattenförmigen Abschnittes 13 des Basisteiles 3 vorliegt, durch den bei Verschiebungen der Lagerungsplatte 1 relativ zu dem Basisteil 3 ein Einquetschen von Hautfalten zwischen der Lagerungsplatte 1 und dem plattenförmigen Abschnitt 13 vermieden ist.

Die Fig. 1 zeigt in Verbindung mit der Fig. 3, daß der plattenförmige Abschnitt des Basisteiles 3 zwei Plattenteile 13a, 13b aufweist, die in Richtung der Längsachse des Patientenlagerungstisches und damit in Verschieberichtung gesehen auf unterschiedlichen Seiten der Ausnehmung 14 des Basisteiles 3 angeordnet sind. Die Plattenteile 13a, 13b, deren einander zugewandte Kanten 52, 53 die Ausnehmung 14 begrenzen, sind durch einen Tragholm 18 miteinander verbunden. Der Tragholm 18 verläuft unterhalb des Bereiches 10 der Lagerungsplatte 1 entlang deren von der Ausnehmung 9 unberührten, also der in Fig. 1 hinteren, Längskante. Das Plattenteil 13b ist mit dem Basisteil 3 verbunden. Der Tragholm 18 und das an dem Tragholm 18 angebrachte Plattenteil 13a bilden also eine wirksame Unterstützung der durch die Ausnehmung 9 geschwächten Lagerungsplatte 1 im Bereich ihres freien Endes. Das Plattenteil 13a wirkt als an dem freien Ende des Tragholmes 18 angebrachter, sich quer zur Längsachse der Lagerungsplatte 1 erstreckender Tragarm, der Verwindungen der Lagerungsplatte 1 entgegenwirkt.

Anhand der Fig. 4 wird deutlich, daß das Plattenteil 13a an seinem zur Befestigung an dem Tragholm 18 vorgesehenen Ende einen etwa U-förmigen Abschnitt 19 aufweist, der den Tragholm 18, der einen etwa rechteckigen Querschnitt besitzt, von oben umgreift. Der äußere Schenkel 20 des U-förmigen Abschnittes 19 ist mit Langlöchern 21 versehen, in die bei an dem Tragholm 18 angebrachtem Plattenteil 13a an der äußeren Seitenfläche des Tragholmes 18 angebrachte Vorsprünge 22 eingreifen. Dabei liegen die Vorsprünge 22 an den unteren Begrenzungskanten der Langlöcher 21 an. Das Plattenteil 13a kann also von dem Tragholm getrennt werden, indem es an seinem freien Ende so weit angehoben wird, daß der innere Schenkel 23 seines U-förmigen Abschnittes 19 nicht mehr mit der inneren Seitenfläche des Tragholmes 18 in Eingriff steht, und indem es dann nach außen abgenommen wird. In entsprechender Weise kann das Plattenteil 13a auch wieder an dem Tragholm 18 angebracht werden. Es versteht sich, daß zum Abnehmen bzw. Anbringen des Plattenteiles 13a die Lagerungsplatte 1 von dem Basisteil 3 getrennt werden muß.

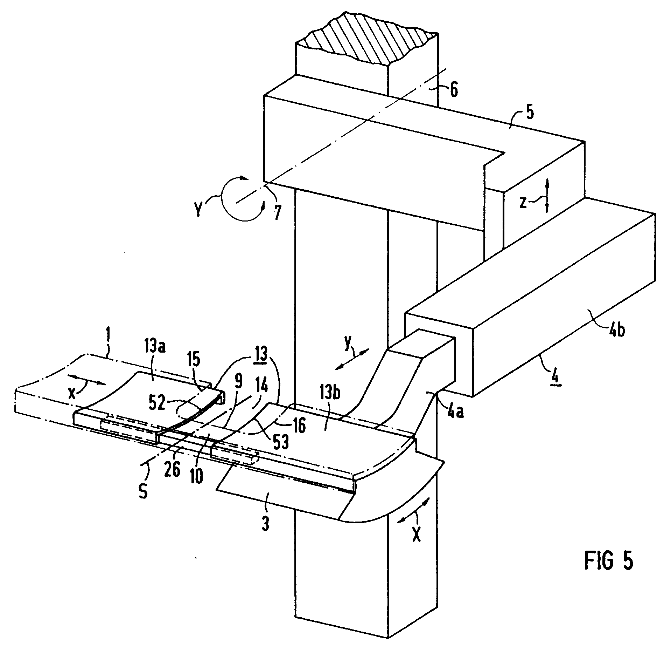

Wie insbesondere die Fig. 3 zeigt, ist der Tragholm 18 in einer entsprechenden Führungsbohrung 24 des Basisteiles 3 aufgenommen. In einer weiteren, auf der anderen Seite des Basisteiles 3 befindlichen Führungsbohrung 25 (siehe Fig. 2 und 5) ist ein weiterer Tragholm 26 aufgenommen. Die Tragholme 18, 26 sind in ihrer jeweiligen Führungsbohrung 24, 25 längsverschieblich geführt, so daß die Möglichkeit besteht, wahlweise einen der Tragholme 18, 26 aus dem Basisteil 3 auszuziehen und das Plattenteil 13a an dem jeweils ausgezogenen Tragholm 18 bzw. 26 anzubringen. Es besteht somit die Möglichkeit, den Patientenlagerungstisch ausgehend von dem Zustand gemäß Fig. 1, in dem sich die Ausnehmungen 9 und 14 in der vorderen Längskante des Patientenlagerungstisches befinden, derart zu verändern, daß sich die Ausnehmungen 9 und 14 gemäß Fig. 5 in der hinteren Längskante des Patientenlagerungstisches befinden. Hierzu ist es lediglich erforderlich, die Lagerungsplatte 1 von dem Basisteil 3 zu trennen, das Plattenteil 13a von dem Tragholm 18 zu trennen, den Tragholm 18 in die Führungsbohrung 24 einzuschieben, den Tragholm 26 aus seiner Führungsbohrung 25 auszuziehen, das Plattenteil 13a an dem Tragholm 26 anzubringen und die gewendete Patientenlagerungsplatte 1 wieder auf das Basisteil 3 aufzusetzen.

Um sicherzustellen, daß sich beim Wenden der Lagerungsplatte 1 die Lage der Ausnehmung 9 der Lagerungsplatte 1 in bezug auf die Längsachse des Patientenlagerungstisches nicht ändert, ist die Lagerungsplatte 1 zu einer quer zur Längsachse des Patientenlagerungstisches und durch die Ausnehmung 9 verlaufende Symmetrieachse S spiegelsymmetrisch ausgebildet.

Um die erwähnte Verstellbarkeit der Lagerungsplatte 1 relativ zu dem Basisteil 3 zu gewährleisten, ist die von der Auflagefläche 2 abgewandte Unterseite 12 der Lagerungsplatte 1 als Eingriffsfläche ausgebildet, die an der ebenfalls als Eingriffsfläche ausgebildeten Oberseite 11 des plattenförmigen Abschnittes 13 des Basisteiles 3 flächenhaft anliegt. Wird die Lagerungsplatte 1 relativ zu dem Basisteil 3 verstellt, gleiten die Unterseite 12 der Lagerungsplatte 1 und die Oberseite 11 des plattenförmigen Abschnittes 13 des Basisteiles 3 aufeinander. Die Lagerungsplatte 1 und der plattenförmige Abschnitt 13 des Basisteiles 3 sind einander nicht nur hinsichtlich ihrer äußeren Abmessungen, d.h. ihrer Länge und Breite, sondern auch in ihrem Querschnitt weitgehend angepaßt, so daß sich zwischen der Unterseite 11 der Lagerungsplatte 1 und der Oberseite 13 des plattenförmigen Abschnittes 13 des Basisteiles 3 eine große Anlagefläche ergibt. Die zwischen den beiden Teilen vorliegende Flächenpressung ist somit nur gering, so daß kein wesentlicher Verschleiß auftreten kann. Ebenfalls im Interesse eines geringen Verschleißes ist die Oberseite 11 des plattenförmigen Abschnittes 13 des Basisteiles 3 durch einen reibungsmindernden Belag 27 gebildet, wobei dann die der Unterseite 12 der Lagerungsplatte 1 zugewandte Seite des Belages 27 die eigentliche Eingriffsfläche des Basisteiles 3 bildet. Der reibungsmindernde Belag 27 bewirkt zugleich, daß für die Verstellung der Lagerungsplatte 1 nur eine geringe Betätigungskraft erforderlich ist. Als Material für den reibungsmindernden Belag ist ein polymerer Werkstoff, nämlich Polyäthylen vorgesehen, wodurch der Belag 27 auf einfache Weise, nämlich durch Kleben, an dem Basisteil 3 befestigt ist. Um die Mutter 56 in der erforderlichen Weise an der Lagerungsplatte 1 befestigen zu können, ist der plattenförmige Abschnitt 13 mit einem Schlitz 61 versehen, der sich durch das Plattenteil 13b und den Belag 27 erstreckt und sich mit dem Schlitz 60 des Basisteiles 3 deckt. Die Breite der Schlitze 60 und 61 ist auf das zum Durchtritt der Mutter 56 erforderliche Maß beschränkt.

Infolge der beschriebenen Ausbildung der Unterseite 12 der Lagerungsplatte 1 und der Oberseite 11 des plattenförmigen Abschnittes 13 des Basisteiles 3 als Eingriffsflächen erfährt die als dünnwandiges schalenförmiges Bauteil ausgebildete Lagerungsplatte 1 eine gleichmäßige Unterstützung, so daß Verwindungen und Durchbiegungen der Lagerungsplatte 1 nicht zu befürchten sind. Die Lagerungsplatte 1 ist aus faserverstärktem Kunststoff, vorzugsweise kohlefaserverstärktem Kunststoff, gebildet. Es ist dann auf einfache Weise möglich, die Unterseite 12 der Lagerungsplatte 1 unmittelbar auf spanlosem Wege bei der Herstellung der Lagerungsplatte 1 als Eingriffsfläche auszubilden. Auch der plattenförmige Abschnitt 13 des Basisteiles 3 ist aus faserverstärktem Kunststoff, vorzugsweise kohlefaserverstärktem Kunststoff, gebildet, so daß auch hier die Oberfläche des plattenförmigen Abschnittes 13 bereits bei dessen Herstellung durch spanlose Formgebung die Form erhält, die erforderlich ist, um nach dem Aufkleben des Belages 27 eine Oberseite 11 zu erhalten, die geeignet ist, um mit der Unterseite 12 der Lagerungsplatte 1 wie beschrieben zusammenwirken zu können.

Die Verstellbarkeit der Lagerungsplatte 1 in Längsrichtung des Patientenlagerungstisches beschränkt sich nicht auf die erwähnten ± 100 mm. Vielmehr ist, um beispielsweise endoskopische Untersuchungen durchführen zu können, eine Verstellbarkeit in Längsrichtung des Patientenlagerungstisches von insgesamt etwa 700 mm gegeben. Aus diesem Grunde ist die gesamte Unterseite 12 der Lagerungsplatte 1 als Eingriffsfläche ausgebildet. Im Falle des dargestellten Ausführungsbeispieles beträgt die Größe der Eingriffsfläche, d.h. der Oberseite 11, des plattenförmigen Abschnittes 13 des Basisteiles 3 etwa 60 % der Fläche der Eingriffsfläche der Lagerungsplatte 1. Da in beiden Extrempositionen, die die Lagerungsplatte 1 in bezug auf das Basisteil 3 einnehmen kann, die gesamte Oberseite 11 des plattenförmigen Abschnittes 13 des Basisteiles 3 an der Unterseite 12 der Lagerungsplatte 1 anliegt, ist stets eine gute Unterstützung der Lagerungsplatte 1 gewährleistet. Für den Fall, daß nicht die gesamte Unterseite 12 der Lagerungsplatte 1 als Eingriffsfläche ausgebildet ist, sollte diese wenigstens 30 % der Fläche der Unterseite 12 der Lagerungsplatte 1 umfassen. Außerdem sollte sichergestellt sein, daß stets wenigstens 50 % der Unterseite 12 der Lagerungsplatte 1 an der Eingriffsfläche des Basisteiles 3 anliegen. Die Eingriffsflächen sowohl der Lagerungsplatte 1 als auch das Basisteiles 3 müssen nicht unbedingt als durchgehende Flächen ausgebildet sein, sondern können in mehrere Abschnitte unterteilt sein, so wie dies beispielsweise im Falle der Oberseite 11 des plattenförmigen Abschnittes 13 des Basisteiles 3 infolge des Umstandes der Fall ist, daß der plattenförmige Abschnitt 13 aus den beiden Plattenteilen 13a, 13b zusammengesetzt ist.

Die Eingriffsflächen sowohl der Lagerungsplatte 1 als auch des plattenförmigen Abschnittes 13 des Basisteiles 3 erstrecken sich jeweils bis an die quer zur Tischlängsrichtung verlaufenden Begrenzungskanten 15, 16 der Ausnehmung 9 bzw. die Begrenzungskanten 52, 53 der Ausnehmung 14. Hierdurch ist die bereits erwähnte im wesentlichen spaltlose Anlage der Begrenzungskanten 15, 16 der Ausnehmung 9 an der Oberseite 11 des plattenförmigen Abschnittes 13 des Basisteiles 3 gewährleistet.

Um eine sichere Führung der Lagerungsplatte 1 relativ zu dem Basisteil 3 quer zur Längsachse des Patientenlagerungstisches, also quer zur Verschieberichtung, zu gewährleisten, ist die Lagerungsplatte an ihren beiden Längskanten mit nach unten abgewinkelten Führungsborden 28, 29 versehen, die mit ihren Innenseiten an den entsprechenden ebenfalls nach unten abgewinkelten Längskanten 58a, 59a bzw. 58b, 59b der Plattenteile 13a und 13b des plattenförmigen Abschnittes 13 des Basisteiles 3 anliegen. Dabei stellen die Innenseiten der Führungsborde 28, 29 Bestandteile der Eingriffsfläche der Lagerungsplatte 1 und die Außenseiten der nach unten abgewinkelten Längskanten 58a, 59a, 58b, 59b der Plattenteile 13a, 13b Bestandteile der Eingriffsfläche des Basisteiles 3 dar. Der Belag 27 bedeckt daher auch die Außenseiten der Längskanten 58a, 59a, 58b, 59b. Der an der mit der Ausnehmung 9 versehenen Längskante der Lagerungsplatte 1 angebrachte Führungsbord 28 weist im Bereich der Ausnehmung eine Unterbrechung auf. Die Führungsborde 28, 29 sind an den Innenseiten ihrer freien Enden mit Leisten 30, 31 versehen, die von unten an den abgewinkelten Längskanten der Plattenteile 13a, 13b des plattenförmigen Abschnittes 13 des Basisteiles 3 anliegen, wobei die Leiste 30 im Bereich der Ausnehmung 9 der Lagerungsplatte 1 unterbrochen ist. Es ist so ein sicherer Zusammenhalt der Lagerungsplatte 1 mit dem Basisteil 3 insbesondere auch beim Schwenken des Patientenlagerungstisches in Richtung des gekrümmten Doppelpfeiles X und/oder des gekrümmten Doppelpfeiles Y gewährleistet.

Um die Patientenlagerungsplatte in der zuvor beschriebenen Weise wenden zu können, muß diese also von der zur Verschiebung der Lagerungsplatte 1 in Richtung des Doppelpfeiles x dienenden Antriebseinrichtung 54, 55, 56 getrennt werden und in Richtung der Längsachse des Patientenlagerungstisches von dem Basisteil 3 abgezogen werden. Die gewendete Lagerungsplatte 1 wird anschließend in umgekehrter Richtung wieder auf das Basisteil 3 aufgeschoben und mit der erwähnten Antriebseinrichtung gekoppelt. Zur Trennung der Antriebseinrichtung 54, 55, 56 von der Lagerungsplatte 1 wird die Mutter 56 von der Lagerungsplatte 1 gelöst, indem eine nicht dargestellte, von der Oberseite der Lagerungsplatte her zugängliche Schraube gelöst wird, die zur Befestigung der Mutter 56 an der Lagerungsplatte 1 dient.

Die Lagerungsplatte 1 ist mit entlang ihren Längskanten verlaufenden Schienen 32, 33 kreisförmigen Querschnittes versehen, die zur Befestigung von Zubehörteilen dienen. Dabei ist die an der mit der Ausnehmung 9 versehenen Längskante der Lagerungsplatte 1 angebrachte Schiene 32 im Bereich der Ausnehmung 9 unterbrochen. Die Schienen 32, 33 dienen zur Befestigung von Zubehörteilen, so wie dies in Fig. 1 am Beispiel eines Infusions-Ständers 34 und einer Fußbank 35 angedeutet ist. Der Infusions-Ständer 34 und die Fußbank 35 sind mit geeigneten Klemmvorrichtungen versehen, die eine Befestigung dieser Teile an einer gewünschten Position längs der Lagerungsplatte 1 gestatten.

Wie aus der Fig. 1 ersichtlich ist, besteht die Möglichkeit, die Ausnehmung 9 der Lagerungsplatte 1 mittels eines Verschlußteiles 36, das von den Plattenteilen 13a und 13b des plattenförmigen Abschnittes 13 des Basisteiles 3 unterstützt wird, zu verschließen, indem das Verschlußstück 36 in die Ausnehmung 9 eingelegt wird. Das Verschlußstück 36 ist mit einer Polsterauflage 37 versehen, deren Dicke der der Polsterauflage 17 entspricht.

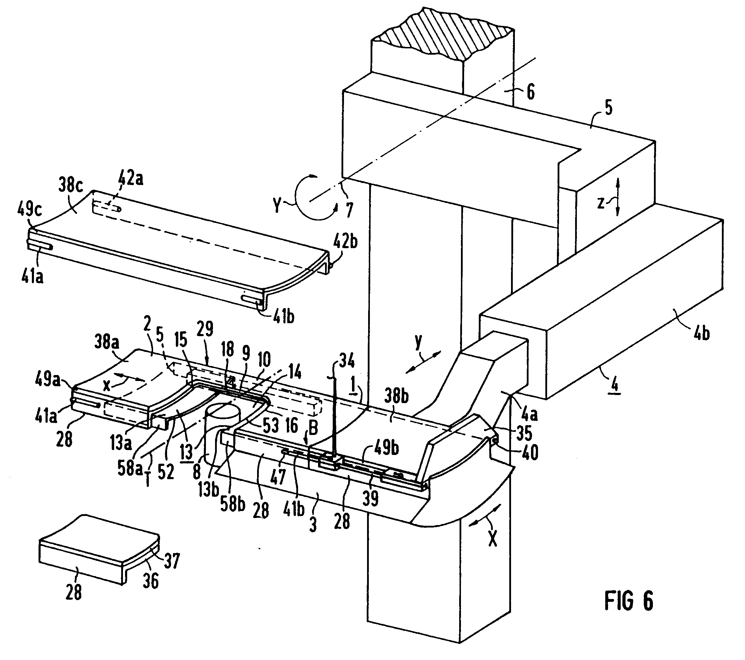

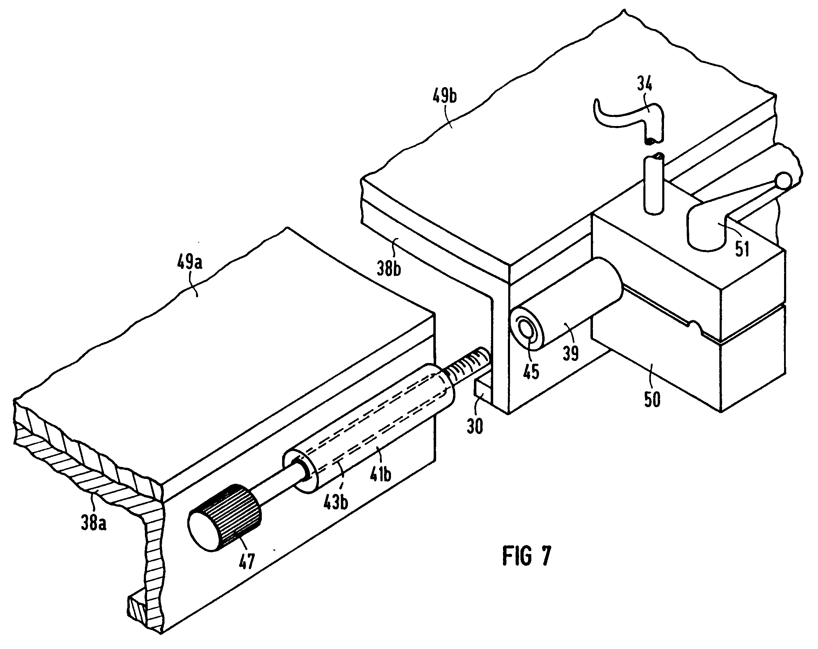

In den Fig. 6 und 7 ist ein zweites Ausführungsbeispiel eines erfindungsgemäßen Patientenlagerungstisches dargestellt, das sich von dem zuvor beschriebenen Ausführungsbeispiel lediglich durch die Ausbildung der Lagerungsplatte 1 unterscheidet, weshalb in den Fig. 6 und 7 für gleiche Teile die gleichen Bezugszeichen wie in den vorangegangenen Figuren verwendet werden. Im Falle der Fig. 6 ist die Lagerungsplatte 1 zweiteilig ausgeführt und weist die miteinander verbundenen Plattenabschnitte 38a und 38b auf. Dabei ist der das freie Ende des Patientenlagerungstisches bildende Plattenabschnitt 38a mit der Ausnehmung 9 versehen. Die nicht dargestellte zur Verschiebung der Lagerungsplatte 1 in Richtung des Doppelpfeiles x dienende Antriebseinrichtung greift mit ihrer Mutter an dem an dem Basisteil 3 angebrachten Plattenabschnitt 38b an. Der Plattenabschnitt 38b ist mit Schienen 39, 40 versehen, die entlang seiner Längskanten verlaufen und einen kreisförmigen Querschnitt aufweisen. Die Schienen 39, 40 dienen zur Befestigung von Zubehörteilen, z.B. eines Infusions-Ständers 34, sowie zur Befestigung der Fußbank 35. Im Bereich der Enden des Plattenabschnittes 38a ist dieser an seinen Längskanten 28, 29 mit Schienenabschnitten versehen, von denen in Fig. 6 nur die Schienenabschnitte 41a, 41b sichtbar sind. Die Schienenabschnitte besitzen, so wie Fig. 7 dies für den Schienenabschnitt 41b anhand der Bohrung 43b zeigt, axial durchgehende Bohrungen. An ihren von dem Basisteil 3 abgewandten Enden sind die Schienen 39, 40 in der aus Fig. 7 am Beispiel der Schiene 39 und der Gewindebohrung 45 ersichtlichen Weise mit Gewindebohrungen versehen, die bei auf dem plattenförmigen Abschnitt 13 des Basisteiles 3 aufgesetztem Plattenabschnitt 38a mit den Bohrungen der Schienenabschnitte fluchten. Wie dies aus Fig. 7 am Beispiel des Schienenabschnittes 41b mit der Bohrung 43b, der Schiene 39 mit der Gewindebohrung 45 und der Rändelschraube 47 ersichtlich ist, sind die beiden Plattenabschnitte 38a, 38b der Lagerungsplatte 1 mit Hilfe von Rändelschrauben, die sich durch die Bohrungen der Schienenabschnitte erstrecken und in die Gewindebohrungen der Schienen 39, 40 einschraubbar sind, lösbar miteinander verbunden.

Werden die Rändelschrauben entfernt, kann der Plattenabschnitt 38a axial von dem plattenförmigen Abschnitt 13 des Basisteiles 3 abgezogen, gewendet und wieder auf den plattenförmigen Abschnitt 13 des Basisteiles 3 aufgeschoben werden, so daß sich die in dem Plattenabschnitt 38a der Lagerungsplatte 1 befindliche Ausnehmung 9 wahlweise in der hinteren oder der vorderen Längskante der Lagerungsplatte 1 befinden kann. Es versteht sich, daß dann jeweils nur der geeignete Tragholm 18 bzw. 26 aus dem Basisteil 3 ausgezogen und mit dem Plattenteil 13a versehen ist. Im Gegensatz zu dem zuvor beschriebenen Ausführungsbeispiel erübrigt sich infolge der Teilung der Lagerungsplatte 1 in die zwei Plattenabschnitte 38a, 38b beim Wenden des Plattenabschnittes 38a die Trennung der erwähnten Antriebseinrichtung von der Lagerungsplatte 1. Um sicherzustellen, daß sich beim Wenden des Plattenabschnittes 38a die Lage der Ausnehmung 9 in bezug auf die Längsachse des Patientenlagerungstisches nicht ändert, ist der Plattenabschnitt 38a ähnlich wie im Falle des zuvor beschriebenen Ausführungsbeispieles die Lagerungsplatte 1 zu einer quer zur Längsachse des Patientenlagerungsrungstisches und durch die Ausnehmung 9 verlaufenden Symmetrieachse T spiegelsymmetrisch ausgebildet.

Ähnlich wie im Falle des zuvor beschriebenen Ausführungsbeispieles besteht die Möglichkeit, die Ausnehmung 9 des Plattenabschnittes 38a, beide Plattenabschnitte 38a und 38b sind übrigens mit Polsterauflagen 49a bzw. 49b versehen, durch das mit der Polsterauflage 37 versehene Verschlußstück 36 zu verschließen. Außerdem besteht, so wie dies in Fig. 6 angedeutet ist, für Behandlungsfälle, die dies gestatten, die Möglichkeit, anstelle des mit der Ausnehmung 9 versehenen Plattenabschnittes 38a einen ohne Ausnehmung ausgeführten Plattenabschnitt 38c mit dem Plattenabschnitt 38b zu verbinden. Der Plattenabschnitt 38c ist mit einer Polsterauflage 49c versehen. Im Falle des Plattenabschnittes 38c sind übrigens sämtliche vier Schienenabschnitte 41a, 41b, 42a, 42b dargestellt.

Aus der Fig. 7 ist ersichtlich, daß der Infusions-Ständer 34 mit einem klammerartigen Klemmstück 50 versehen ist, das mittels einer Klemmschraube 51 auf der Schiene 39 klemmbar ist. Die Befestigung der Fußbank 35 erfolgt in entsprechender Weise (nicht dargestellt). Auch die Befestigung des Infusionsständers 34 und der Fußbank 35 im Falle des zuerst beschriebenen Ausführungsbeispieles an den Schienen 32, 33 erfolgt in der in Fig. 7 verdeutlichten Weise.

Die Ausführungsbeispiele betreffen ausschließlich Patientenlagerungstische, die Bestandteil eines Arbeitsplatzes zur Behandlung eines Patienten mit fokussierten Stoßwellen zur nicht invasiven Zertrümmerung von im Körper des Patienten befindlichen Konkrementen sind. Es können jedoch auch Röntgenuntersuchungstische, Patientenlagerungstische für die Urologie, Lagerungstische für die Strahlentherapie usw. gemäß der Erfindung ausgebildet werden. Außerdem ist im Falle der Ausführungsbeispiele jeweils nur eine Verstellbarkeit der Lagerungsplatte 1 relativ zu dem Basisteil 3 vorgesehen, die in Richtung der Längsachse des Patientenlagerungstisches und geradlinig erfolgt. Mit den Merkmalen der Erfindung sind jedoch auch andere und/oder zusätzliche Verstellmöglichkeiten realisierbar.

## Patentansprüche

1. Patientenlagerungstisch mit einer Lagerungsplatte (1), welche eine Auflagefläche (2) für einen Patienten aufweist und mit einem Ausschnitt (9) versehen ist, durch den die Körperoberfläche eines auf der Auflagefläche (2) liegenden Patienten zu medizinischen Zwecken zugänglich ist, und mit einem die Lagerungsplatte (1) tragenden Basisteil (3), wobei die Lagerungsplatte (1) relativ zu dem Basisteil (3) in einer Verstellrichtung verstellbar ist,
**dadurch gekennzeichnet,**
a) daß das Basisteil (3) unterhalb der Lagerungsplatte (1) angeordnet ist und eine Oberseite (11) aufweist, die der Unterseite (12) der Lagerungsplatte (1) zugewandt ist,
b) daß das Basisteil (3) eine sich durch dessen Oberseite (11) erstreckende Öffnung (14) aufweist, deren Erstreckung (b) zumindest in Verstellrichtung geringer ist als die entsprechende Erstreckung (a) des Ausschnittes (9) der Lagerungsplatte (1),
c) daß die Lagerungsplatte (1) relativ zu dem Basisteil (2) derart positionierbar ist, daß der Ausschnitt (9) der Lagerungsplatte die Öffnung (14) des Basisteiles (3) freigibt, und
d) daß die Oberseite (11) des Basisteiles (3) innerhalb des Ausschnittes (9) der Lagerungsplatte (1) eine zusätzliche Auflagefläche bildet.

2. Patientenlagerungstisch nach Anspruch 1, **dadurch gekennzeichnet,** daß die Lagerungsplatte (1) wenigstens mit den quer zur Verstellrichtung verlaufenden Begrenzungskanten (15, 16) des Ausschnittes (9) im wesentlichen spaltlos an der Oberseite (11) des Basisteiles (3) anliegt.

3. Patientenlagerungstisch nach Anspruch 1 oder 2, **dadurch gekennzeichnet,** daß die Auflagefläche (2) der Lagerungsplatte (1) mit einer Polsterauflage (17) versehen ist, die bis an die Begrenzungskanten (15, 16) des Ausschnittes (9) reicht.

4. Patientenlagerungstisch nach einem der Ansprüche 1 bis 3, **dadurch gekennzeichnet,** daß die Lagerungsplatte (1) als dünnwandiges Bauteil ausgeführt ist.

5. Patientenlagerungstisch nach einem der Ansprüche 1 bis 4, **dadurch gekennzeichnet,** daß die Lagerungsplatte (1) auf ihrer der Auflagefläche (2) abgewandten Seite (12) eine Eingriffsfläche aufweist, die an einer entsprechend geformten Eingriffsfläche des Basisteiles (3) flächenhaft anliegt, daß die Eingriffsflächen sich zu den quer zur Verstellrichtung verlaufenden Begrenzungskanten (15, 16 bzw. 52, 53) des Ausschnittes (9) bzw. der Öffnung (14) erstrecken, und daß die Eingriffsflächen der Lagerungsplatte (1) und des Basisteiles (3) beim Verstellen der Lagerungsplatte (1) relativ zum Basisteil (3) aufeinander gleiten.

6. Patientenlagerungstisch nach einem der Ansprüche 1 bis 5, **dadurch gekennzeichnet,** daß der Ausschnitt (9) als Ausnehmung in einer der Begrenzungskanten der Lagerungsplatte (1) ausgeführt ist und die Öffnung (14) als Ausnehmung in der entsprechenden Begrenzungskante des Basisteiles (3) ausgeführt ist.

7. Patientenlagerungstisch nach Anspruch 6, **dadurch gekennzeichnet,** daß das Basisteil (3) zwei Plattenteile (13a, 13b) aufweist, die in Verstellrichtung gesehen auf unterschiedlichen Seiten der Öffnung (14) angeordnet sind, und daß die Plattenteile (13a, 13b) durch einen Tragholm (18, 26) miteinander verbunden sind, der sich wenigstens im Bereich der Ausnehmung (9) der Lagerungsplatte (1) entlang der der Ausnehmung (9) der Lagerungsplatte (1) gegenüberliegenden Begrenzungskante der Lagerungsplatte (1) erstreckt.

8. Patientenlagerungstisch nach Anspruch 6 oder 7, **dadurch gekennzeichnet,** daß die Lagerungsplatte (1) an dem Basisteil (3) wahlweise derart anbringbar ist, daß sich die Ausnehmung (9) auf der einen oder der anderen Seite des Patientenlagerungstisches befindet.

9. Patientenlagerungstisch nach Anspruch 6 oder 7, **dadurch gekennzeichnet,** daß die Lagerungsplatte (1) zweiteilig ausgeführt ist, wobei ein Plattenabschnitt (38a) die Ausnehmung (9) aufweist und mit dem anderen Plattenabschnitt (38b) wahlweise derart verbindbar ist, daß sich die Ausnehmung (9) auf der einen oder der anderen Seite des Patientenlagerungstisches befindet.

10. Patientenlagerungstisch nach Anspruch 8 oder 9, **dadurch gekennzeichnet,** daß zwei Tragholme (18, 26) vorgesehen sind, die in Abhängigkeit von der Position der Ausnehmung (9) der Lagerungsplatte (1) auf der einen oder der anderen Seite des Patientenlagerungstisches wahlweise ausziehbar sind.

## Claims

1. Patient support table having a support plate (1) which has a seating surface (2) for a patient and is provided with a cut-out (9) through which the body surface of a patient lying on the seating surface (2) is accessible for medical purposes, and having a base part (3) which carries the support plate (1), wherein the support plate (1) can be adjusted in one direction of adjustment relative to the base part (3), characterised in that
(a) the base part (3) is arranged beneath the support plate (1) and has an upper side (11) which faces towards the underside (12) of the support plate (1),
(b) the base part (3) has an opening (14) extending through the upper side (11) thereof whose extent (b), at least in the direction of adjustment, is less than the corresponding extent (a) of the cut-out (9) of the support plate (1),
(c) the support plate (1) can be positioned relative to the base part (2) (sic) so that the cut-out (9) of the support plate exposes the opening (14) in the base part (3), and
(d) the upper side (11) of the base part (3) forms an additional seating surface within the cut-out (9) of the support plate (1).

2. Patient support table according to claim 1, characterised in that at least the boundary edges (15, 16) of the cut-out (9) of the support plate (1), which extend transversely to the direction of adjustment, bear in a substantially gap-free manner against the upper side (11) of the base part (3).

3. Patient support table according to claim 1 or 2, characterised in that the seating surface (2) of the support plate (1) is provided with a cushion seating (17) which extends up to the boundary edges (15, 16) of the cut-out (9).

4. Patient support table according to one of claims 1 to 3, characterised in that the support plate (1) is constructed as a thin-walled component.

5. Patient support table according to one of claims 1 to 4, characterised in that the side (12) of the support plate (1) facing away from the seating surface (2) has a surface of action which bears flatly against a correspondingly shaped surface of action of the base part (3), in that the surfaces of action extend to the boundary surfaces (15, 16 and 52, 53, respectively) of the cut-out (9) and the opening (14), respectively, which boundary surfaces extend transversely to the direction of adjustment, and in that the surfaces of action of the support plate (1) and the base part (3) slide on one another when the support plate (1) is adjusted relative to the base part (3).

6. Patient support table according to claims 1 to 5, characterised in that the cut-out (9) is formed as a recess in one of the boundary edges of the support plate (1) and the opening (14) is formed as a recess in the corresponding boundary edge of the base part (3).

7. Patient support table according to claim 6, characterised in that the base part (3) has two plate parts (13a, 13b) which are arranged on different sides of the opening (14), seen in the direction of adjustment, and in that the plate parts (13a, 13b) are connected together by a carrying spar (18, 26) which extends at least in the region of the recess (9) of the support plate (1) along the boundary edge of the support plate (1) located opposite the recess (9) of the support plate (1).

8. Patient support table according to claim 6 or 7, characterised in that the support plate (1) can be selectively attached to the base part (3) in such a way that the recess (9) is located on either side of the patient support table.

9. Patient support table according to claim 6 or 7, characterised in that the support plate (1) is constructed in two parts, wherein a plate section (38a) has the recess (9) and can be selectively connected to the other plate section (38b) in such a way that the recess (9) is located on either side of the patient support table.

10. Patient support table according to claim 8 or 9, characterised in that there are provided two carrying spars (18, 26) which can be selectively pulled out, in dependence upon the position of the recess (9) of the support plate (1) on either side of the patient support table.

## Revendications

1. Table de support pour patient comportant un plateau de support (1) qui possède une surface d'appui (2) pour un patient et comporte une découpe (9), à travers laquelle la surface du corps d'un patient allongé sur la surface de support (2) est accessible pour un traitement médical, et comportant une partie de base (3) supportant la table de support (1), le plateau de support (1) étant déplaçable par rapport à la partie de base (3), dans une direction de déplacement,
caractérisée par le fait
a) que la partie de base (3) est disposée au-dessous du plateau de support (1) et possède une face supérieure (11), qui est tournée vers la face inférieure (12) du plateau de support (1),
b) que la partie de base (3) possède une ouverture (14) qui traverse la face supérieure (11) de la partie de base et dont l'extension (b) au moins dans la direction de déplacement est inférieure à l'extension correspondante (a) de la découpe (9) du plateau de support (1),
c) que le plateau de support (1) peut être positionné par rapport à la partie de base (2) de telle sorte que la découpe (9) du plateau de support dégage l'ouverture (14) de la partie de base (3), et
d) que la face supérieure (11) de la partie de base (3) forme, à l'intérieur de la découpe (9) du plateau de support (1), une surface d'appui supplémentaire.

2. Table de support pour patient suivant la revendication 1, caractérisée par le fait que le plateau de support (1) s'applique au moins par les bords limites (15,16) de la découpe (9), qui s'étendent transversalement par rapport à la direction de déplacement, essentiellement sans la présence d'aucune fente, contre la face supérieure (11) de la partie de base (3).

3. Table de support pour patient suivant la revendication 1 ou 2, caractérisée par le fait que la surface de support (2) du plateau de support (1) comporte un revêtement de rembourrage (17), qui s'étend jusqu'aux bords limites (15,16) de la découpe (9).

4. Table de support pour patient suivant l'une des revendications 1 à 3, caractérisée par le fait que le plateau de support (1) est réalisé sous la forme d'un composant à paroi mince.

5. Table de support pour patient suivant l'une des revendications 1 à 4, caractérisée par le fait que le plateau de support (1) possède, sur sa face (12) tournée à l'opposé de la surface de support (2), une surface d'engagement qui s'applique sur une certaine étendue contre une surface d'engagement de forme correspondante de la partie de base (3), que les surfaces d'engagement s'étendent jusqu'aux bords limites (15,16,52,53) de la découpe (9) ou de l'ouverture (14), qui s'étendent transversalement par rapport à la direction de déplacement, et que les surfaces d'engagement du plateau de support (1) et de la partie de base (3) glissent l'une sur l'autre lors du déplacement du plateau de support (1) par rapport à la partie de base (3).

6. Table de support pour patient suivant l'une des revendications 1 à 5, caractérisée par le fait que la découpe (9) est réalisée sous la forme d'un évidement ménagé dans l'un des bords limites du plateau de support (1) et que l'ouverture (14) est réalisée sous la forme d'un évidement ménagé dans le bord limite correspondant de la partie de base (3).

7. Table de support pour patient suivant la revendication 6, caractérisée par le fait que la partie de base (3) possède deux éléments de plaque (13a,13b), qui sont disposés sur des côtés différents de l'ouverture (14), lorsqu'on regarde dans la direction de déplacement et que les éléments de plaque (13a,13b) sont reliés entre eux par un longeron (18,26), qui s'étend, au moins dans la zone de l'évidement (9) du plateau de support (1), le long du bord limite du plateau de support (1), qui est situé à l'opposé de l'ouverture (9) du plateau de support (1).

8. Table de support pour patient suivant la revendication 6 ou 7, caractérisé par le fait que le plateau de support (1) peut être monté sur la partie de base (3), au choix, de manière que l'évidement (9) soit situé d'un côté ou de l'autre de la table de support pour patient.

9. Plateau de support pour patient suivant la revendication 7, caractérisé par le fait que le plateau de support (1) est réalisé en deux parties, une partie (38a) du plateau possédant la découpe ou évidement (9), tandis que l'autre partie (38b) du plateau peut être reliée au choix de telle sorte que l'évidement (9) est situé d'un côté ou de l'autre de la table de support pour patient.

10. Plateau de support pour patient suivant la revendication 8 ou 9, caractérisé par le fait qu'il est prévu deux longerons (18,26) qui peuvent être dégagés au choix d'un côté ou de l'autre de la table de support pour patient, en fonction de la position de l'évidement (9) du plateau de support (1).
